# EUROPEAN PATENT APPLICATION

(11) **EP 4 260 858 A1**
(43) Date of publication of application: **18.10.2023**
(21) Application number: 22168540.7
(22) Date of filing: 14.04.2022
(51) Int. Cl.: A61K 31/515, A61K 45/06, A61P 9/00, A61P 25/00, A61P 29/00, A61P 37/00, A61K 9/08, A61K 47/10

(54) **COMPOSITION COMPRISING PRIMIDONE AND PEG**

(71) Applicant: Christian-Albrechts-Universität zu Kiel, 24118 Kiel (DE)
(72) Inventor: Krautwald, Stefan, 24220 Flintbek (DE); Kunzendorf, Ulrich, 24105 Kiel (DE)
(74) Representative: Uexküll & Stolberg

(57) **Abstract**

The present invention relates to a pharmaceutical composition comprising primidone or a pharmaceutically acceptable active metabolite, derivative, salt or solvate thereof in combination with a low-molecular weight polyethylene glycol (PEG) having an average molecular weight of about 180-420 g/mol. The composition is suitable for use in the treatment of diseases or conditions that involve a pathologic level of RIPK1-dependent cell death. Specifically, the composition is suitable for use in the treatment of reperfusion injury disease, a systemic inflammatory disease, a transplantation-related disease, a neurodegenerative disease, an autoimmune disease, an ophthalmologic disease, or a pulmonary disease.

## Description

### COMPOSITION COMPRISING PRIMIDION AND PEG

The present invention relates to a pharmaceutical composition comprising primidone or a pharmaceutically acceptable active metabolite, derivative, salt or solvate thereof in combination with a low-molecular weight polyethylene glycol (PEG) having an average molecular weight of about 180-420 g/mol. The composition is suitable for use in the treatment of diseases or conditions that involve a pathologic level of RIPK1-dependent cell death. Specifically, the composition is suitable for use in the treatment of reperfusion injury disease, a systemic inflammatory disease, a transplantation-related disease, a neurodegenerative disease, an autoimmune disease, an ophthalmologic disease, or a pulmonary disease.

### BACKGROUND OF THE INVENTION

Necroptosis, a regulated form of necrosis, is a caspase-independent programmed cell death mechanism. In necroptosis, a cascade of interactions between the receptor-interacting protein kinase 1 (RIPK1), the receptor-interacting protein kinase 3 (RIPK3) and the mixed lineage kinase domain-like protein (MLKL) protein leads to the formation of a specialized death complex named the necrosome, which triggers MLKL-mediated disruption of the cell membrane and necroptotic cell death (Cho et al., 2009; Sun et al., 2012). The necroptotic cell death leads to the release of cellular content which then triggers an inflammatory response.

The signaling pathways of various subtypes of regulated cell death have been analysed in detail in recent years. It is assumed today that the receptor-interacting protein kinase 1 (RIPK1) plays a crucial role in the activation of regulated cell death. It has therefore been suggested that the inhibition of this protein might offer therapeutic benefit for a number of diseases which are characterized by a pathogenic level of apoptosis and necroptosis, such as ischemia-reperfusion injury, myocardial infarction, acute kidney injury, neurodegenerative disorders like Parkinson's and Alzheimer's disease as well as stroke, sepsis and cancer (Liu & Lalaoui, 2021; Degterev et al. 2019).

WO 2021/048162 A1 shows that primidone and its metabolites and derivatives have been reported to be potential inhibitors of RIPK1 that can be used as active ingredients for treating a number of RIPK1-related diseases. Primidone is an anticonvulsant of the barbiturate class that has long been approved for therapeutic treatment of seizures, including partial and generalized seizures, and tremors. It is sold, amongst others, under the trade names Lepsiral^{®}, Mysoline^{®}, Resimatil^{®}, and Liskantin^{®}. Primidone is also known as desoxyphenobarbital or desoxyphenobarbitone. The IUPAC name of primidone is 5-Ethyl-5-phenyl-1,3-diazinane-4,6-dione. It was found that primidone prevents the activation of RIPK1 that normally occurs by phosphorylation or autophosphorylation. In the absence of phosphorylated RIPK1, the assembly of the necrosome consisting of the proteins RIPK1, RIPK3 and MLKL cannot occur. This ultimately leads to the prevention of RIPK1-mediated cell death.

However, it has also been shown that the solubility of primidone and its metabolites and derivatives in water and in most organic solvents is low. For example, primidone is soluble in water only up to a concentration of about 0.5 mg/ml. In ethanol, primidone is soluble up to a concentration of about 3 mg/ml. In order to provide a therapeutic effect in patients, the concentration of primidone in a pharmaceutical composition to be administered by injection or infusion must be at least 10 mg/ml. While primidone is soluble in dimethyl sulfoxide (DMSO) in amounts of up to 44 mg/ml, DMSO has been reported to be toxic even in low doses and is thus not suitable for use in humans. Apart from low solubility, it has been found to be challenging to identify solvents that provide for compositions in which primidone is stable and does not precipitate upon storage.

Against this background, it is an objective of the present invention to provide a stable and therapeutically effective pharmaceutical composition that comprises primidone or a pharmaceutically acceptable active metabolite, derivative, salt or solvate thereof. According to the invention, this objective is achieved by the pharmaceutical composition defined in the attached claims.

### DESCRIPTION OF THE INVENTION

The present invention is based on the insight that compositions with high concentrations of primidone and improved stability can be obtained by dissolving primidone in a low-molecular PEG. When using primidone as a solvent or co-solvent, high amount of primidone can be solubilised and precipitation upon storage can be avoided. In a first aspect, the present invention therefore relates to a pharmaceutical composition comprising or consisting of:
(i) primidone or a pharmaceutically acceptable active metabolite, derivative, salt or solvate thereof, and more preferably primidone
(ii) a low-molecular weight PEG having an average molecular weight of 180-420 g/mol, and.
(iii) optionally, an aqueous pharmaceutical carrier.

The pharmaceutical composition of the invention is useful for delivery of primidone or a pharmaceutically acceptable active metabolite, derivative, salt or solvate thereof to a patient. In the following, the term "primidone compound" is used to collectively refer both to primidone and its pharmaceutically acceptable active metabolites, derivatives, salts or solvates. The pharmaceutical composition of the invention comprises, apart from the primidone compound, PEG as a solvent or co-solvent in which the primidone compound is dissolved. Preferably, the primidone compound is completely dissolved in PEG. While it is possible that the pharmaceutical composition of the invention comprises only PEG as a pharmaceutically acceptable carrier (i.e. PEG is used as a solvent for the primidone), it is more preferred that the composition comprises PEG and an aqueous pharmaceutical carrier, such as water.

As a first component, the pharmaceutical composition of the invention comprises primidone or a pharmaceutically acceptable active metabolite, derivative, salt or solvate thereof as an active ingredient. As used herein, primidone refers to the compound 5-Ethyl-5-phenyl-1,3-diazinane-4,6-dione having the following structural formula (I):

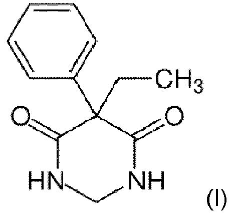

Also included according to the invention are derivatives of the compound of the above formula (I) which are substituted or otherwise modified at one or several positions, as long as these modifications neither substantially affect the inhibitory effect of primidone on the RIPK1 activity nor lead to adverse results in terms of toxicity. For example, one or more hydrogen atoms of the C-H bonds of the heterocyclic ring systems can be substituted by halogen atoms, such as chlorine, bromine or iodine atoms. Further, the hydrogen of the C-H bonds can also be replaced by a short-chained alkyl group such as methyl, ethyl, propyl, or a long-chained alkyl group.

Active metabolites of primidone may also be used in the pharmaceutical composition of the invention. In particular, the composition may comprise the primidone metabolite phenobarbital (PB), also known as phenobarbitone or phenobarb. As used herein, phenobarbital refers to the compound 5-Ethyl-5-phenyl-1,3-diazinane-2,4,6-trione having the following structural formula (II):

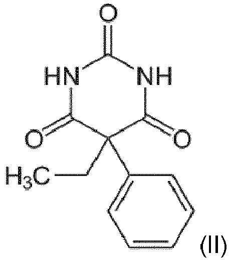

The composition of the invention may also comprise the primidone metabolite phenylethyl-malonamide (PEMA). As used herein, PEMA refers to the compound 2-Ethyl-2-phenyl-malonamide having the following structural formula:

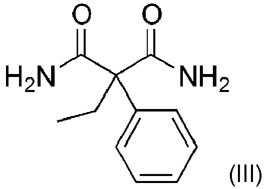

Also included according to the invention are derivatives of the above-depicted metabolite structures (II) and (III) which are substituted or otherwise modified at one or several positions, as long as these modifications neither substantially affect the inhibitory effect of the primidone metabolite on the RIPK1 activity nor lead to adverse results in terms of toxicity. For example, one or more hydrogen atoms of the C-H bonds of the heterocyclic ring systems can be substituted by halogen atoms, such as chlorine, bromine or iodine atoms. Further, the hydrogen of the C-H bonds can also be replaced by a short-chained alkyl group such as methyl, ethyl, propyl, or a long-chained alkyl group.

Pharmaceutically acceptable salts of primidone or its active metabolites or derivatives can also be used in the pharmaceutical composition of the invention. The term "pharmaceutically acceptable salt" refers to non-toxic acid addition salts, alkali metal and alkaline earth metal salts, respectively. Exemplary acid addition salts include hydrochloride, hydrobromide, sulfate, bisulfate, acetate, oxalate, phosphate, citrate, maleate, fumarate, succinate, tartrate and lauryl sulfate. Exemplary alkali metal or alkaline earth metal salts include sodium, potassium, calcium and magnesium salts. In addition, ammonium salts and salts with organic amines can be used as well. Apart from the calcium salt, any other pharmaceutically acceptable cationic salt can be used. The salts are, for example, salts that are obtained by reaction of the free acid form of primidone with a suitable base, such as sodium hydroxide, sodium methoxide, sodium hydride, potassium methoxide, magnesium hydroxide, calcium hydroxide, choline, diethanolamine, and others which are known in the prior art. Solvates of primidone or its metabolites are also part of the present invention. Solvates occur upon the addition of one or more solvent molecules to primidone or its metabolites. If the solvent is water, said addition is a hydration. Solvates of the contemplated active ingredient compound can be held together by ionic bonds and/or covalent bonds.

It is particularly preferred that the pharmaceutical composition of the invention comprises primidone or a pharmaceutically acceptable salt or solvate thereof.

As a second component, the pharmaceutical composition of the invention comprises a low-molecular weight PEG. Within the composition of the invention, PEG serves as a solvent or co-solvent to dissolve the primidone compound. As used herein, the term "low-molecular weight PEG" is considered to refer to a PEG that has an average molecular weight of less than 500 g/mol. These types of PEG are liquid at room temperature and can be used for solubilising the poorly water-soluble primidone into an aqueous composition. The PEG used in the compositions of the invention has an average molecular weight of about 180-420 g/mol. Preferably, the low-molecular weight PEG used in the composition of the invention preferably has an average molecular weight of between about 190-410 g/mol, between about 200-400 g/mol, between about 210-390 g/mol, between about 220-380 g/mol, between about 230-370 g/mol, between about 240-360 g/mol, between about 250-350 g/mol, between about 260-340 g/mol, between about 270-330 g/mol, between about 280-320 g/mol, or between about 290-310 g/mol. It is particularly preferred that the low-molecular weight PEG used in the pharmaceutical composition of the invention has an average molecular weight of between about 280-320 g/mol, and more particularly 280-320 g/mol. Suitable PEG preparations with the desired average molecular weight can be obtained from several different manufacturers, such as from Merck (Darmstadt, Germany), from Biozol, (Eching, Germany), or from Carl Roth GmbH + Co. KG (Karlsruhe, Germany).

Stated differently, it is preferred to use a PEG in the compositions of the invention that has an average molecular weight of less than about 420 g/mol, less than about 410 g/mol, less than about 400 g/mol, less than about 390 g/mol, less than about 380 g/mol, less than about 370 g/mol, less than about 360 g/mol, less than about 350 g/mol, less than about 340 g/mol, less than about 330 g/mol, less than about 320 g/mol, or less than about 310 g/mol. In a particularly preferred aspect, the PEG used in the pharmaceutical composition of the invention has an average molecular weight of about 300 g/mol.

In a preferred embodiment, primidone or the pharmaceutically acceptable active metabolite, derivative, salt or solvate thereof is the only active ingredient in the composition. In another preferred embodiment, the pharmaceutical composition of the invention comprises the primidone compound in combination with another active ingredient, more preferably an antiinflammatory agent or an inhibitor of apoptosis or necroptosis, such as GSK3145095 of GlaxoSmithKline or DNL747 of Sanofi.

The pharmaceutical composition of the invention is preferably formulated for administration to a subject, preferably a human subject. The compositions can be formulated in accordance with standard methods using commonly known excipients. Such methods and suitable excipients are described, for example, in "Remington: The Science and Practice of Pharmacy", Lippincott Williams & Wilkins; 21st ed. (2005). The pharmaceutical composition comprising the primidone or the metabolite, derivative, salt or solvate thereof may be formulated, for example, as a composition for oral, rectal, nasal or parental (including subcutaneous, intramuscular, intravenous and intradermal) administration. Depending on the target tissue to be treated, the compositions can occur in the form of granules, powders, tablets, capsules, syrup, suppositories, injection solutions, emulsions or suspensions.

The pharmaceutical composition may comprise the primidone compound dissolved in PEG, i.e. PEG serves as pharmaceutical carrier for the primidone compound. The pharmaceutical composition may however additionally comprise an aqueous pharmaceutically acceptable carrier which is physiologically with the primidone compound, e.g. water or an aqueous buffer. In such embodiments, water or the aqueous buffer is the pharmaceutically acceptable carrier and PEG is the co-solvent for dissolving the primidone compound into the water or an aqueous buffer.

In a preferred embodiment, pharmaceutical composition of the invention comprises a primidone compound, a PEG having an average molecular weight of 180-420 g/mol, an aqueous pharmaceutical carrier. In another preferred embodiment, pharmaceutical composition of the invention comprises a primidone compound, a PEG having an average molecular weight of 180-420 g/mol, water as an aqueous pharmaceutical carrier. The PEG is preferably present in the composition in an amount of 1-50% (w/w), based on the total weight of the composition. More preferably PEG is present in the composition in an amount of 1-45% (w/w), 1-40% (w/w), 1-35% (w/w), 1-30% (w/w), 1-25% (w/w), 1-20% (w/w), 1-15% (w/w) or 1-10% (w/w), based on the total weight of the composition. The PEG may be present in the composition in an amount of 5-45% (w/w), 5-40% (w/w), 5-35% (w/w), 5-30% (w/w), 5-25% (w/w), 5-20% (w/w), 5-15% (w/w) or 5-10% (w/w), based on the total weight of the composition.

In one preferred embodiment, the pharmaceutical composition of the invention is formulated for oral, buccal, or sublingual administration, and preferably for oral administration. Pharmaceutical compositions for oral, buccal, or sublingual administration may be provided as liquid formulations, for example, as emulsions, syrups, suspensions or solutions. These formulations can be prepared by admixing primidone or its active metabolite, derivative, salt or solvate with the low-molecular weight PEG and optionally with one or more liquid carriers like oils, alcohols, water or combinations thereof. If such liquid carriers are used, they will be used in amounts that do not interfere with the pharmaceutical effect of the primidone compound. Oils which are suitable for being used in this context comprise, for example, olive oil, sesame oil, peanut oil, rape oil, and corn oil. Suitable alcohols comprise ethanol, isopropyl alcohol, hexadecyl alcohol, glycerol and propylene glycol. If the pharmaceutical composition is formulated in the form of a suspension, a fatty acid ester, such as ethyl oleate or isopropyl myristate, a fatty acid glyceride, or an acetylated fatty acid glyceride may be added. The pharmaceutical composition of the invention may also include further excipients, such as binders, diluents, dyes, or the like. The preparation of liquid compositions for oral, buccal, or sublingual administration is discussed in "Remington: The Science and Practice of Pharmacy", Lippincott Williams & Wilkins; 21^{st} ed. (2005).

In another preferred embodiment, the pharmaceutical composition of the invention is formulated for parenteral administration, preferably by injection, e.g. by bolus injection or by continuous infusion. Compositions suitable for injection typically comprise an aqueous solution, an aqueous suspension or an oil suspension which has been prepared by use of the low-molecular weight PEG discussed above. Injectable compositions may also comprise stabilizers or surfactants that can optionally be added to these formulations. Injectable compositions may be formulated in the form of powders that can be reconstituted in a suitable solvent prior to use. Examples include, amongst others, lyophilized or spray-dried powders.

The amount of the pharmaceutical composition which is administered to the patient will ultimately depend on the amount of primidone or an active metabolite, derivative, salt or solvate thereof that is to be delivered to the patient. Factors that influence the amount of primidone to be administered include the type and severity of the disease, age, body weight, sex and overall state of health of the patient to be treated, the simultaneous administration of other therapeutic agents, and the like. The specific amount of the primidone compound to be administered will be determined by the skilled person by using routine methods.

Typical amounts of primidone for delivery to a human patient are in the range of 1 to 50 mg primidone per kilogram body weight of the patient. For children, primidone is typically administered in an amount of 1 to 30 mg primidone compound per kilogram body weight, preferably 5 to 20 mg primidone per kilogram body weight, and more preferably 10 to 20 mg primidone per kilogram body weight. For adults, primidone is typically administered in an amount of 1 to 20 mg the primidone per kilogram body weight, preferably 5 to 15 mg the primidone per kilogram body weight, and more preferably 10 to 15 mg primidone per kilogram body weight. The amounts recited for primidone apply vice versa to the active metabolites, derivatives, salts or solvates of primidone.

The overall daily dosis of primidone for delivery to a human patient typically is in the range of 50 to 5000 mg, preferably in the range of 100 mg to 2500 mg, in the range of 250 mg to 2000 mg, and most preferably in the range of 500 mg to 1500 mg. The doses recited for primidone apply vice versa to the active metabolites, derivatives, salts or solvates of primidone.

It is preferred that primidone is administered in an amount that the concentration of primidone in the plasma is between 0.1 µg/ml and 12 µg/ml, preferably between 0.5 µg/ml and 10 µg/ml, more preferably between 1 µg/ml and 7.5 µg/ml, and even more preferably between 2 µg/ml and 5 µg/ml. The therapy can be monitored and adapted accordingly to provide for the desired plasma concentrations. The concentrations recited for primidone apply vice versa to the active metabolites, derivatives, salts or solvates of primidone.

In a preferred embodiment, the pharmaceutical composition of the invention is formulated as a liquid dosage form for parenteral administration and comprises:
(i) primidone or a pharmaceutically acceptable active metabolite, derivative, salt or solvate thereof in an amount of 50 mg to 5000 mg; and
(ii) a low-molecular weight PEG having an average molecular weight of about 180-420 g/mol, and preferably about 280-320 g/mol.

In another preferred embodiment, the pharmaceutical composition of the invention is formulated as a liquid dosage form for parenteral administration and comprises:
(i) primidone or a pharmaceutically acceptable active metabolite, derivative, salt or solvate thereof in an amount of 250 mg to 2500 mg; and
(ii) a low-molecular weight PEG having an average molecular weight of about 180-420 g/mol, and preferably about 280-320 g/mol.

In another preferred embodiment, the pharmaceutical composition of the invention is formulated as a liquid dosage form for parenteral administration and comprises:
(i) primidone or a pharmaceutically acceptable active metabolite, derivative, salt or solvate thereof in an amount of 50 mg to 5000 mg;
(ii) a low-molecular weight PEG having an average molecular weight of about 180-420 g/mol, and preferably about 280-320 g/mol; and
(iii) an aqueous pharmaceutical carrier.

In another preferred embodiment, the pharmaceutical composition of the invention is formulated as a liquid dosage form for parenteral administration and comprises:
(i) primidone or a pharmaceutically acceptable active metabolite, derivative, salt or solvate thereof in an amount of 250 mg to 2500 mg;
(ii) a low-molecular weight PEG having an average molecular weight of about 180-420 g/mol, and preferably about 280-320 g/mol; and
(iii) an aqueous pharmaceutical carrier.

In another preferred embodiment, the pharmaceutical composition of the invention is formulated as a liquid dosage form for oral administration and comprises:
(i) primidone or a pharmaceutically acceptable active metabolite, derivative, salt or solvate thereof in an amount of 50 mg to 5000 mg; and
(ii) a low-molecular weight PEG having an average molecular weight of about 180-420 g/mol, and preferably about 280-320 g/mol.

In another preferred embodiment, the pharmaceutical composition of the invention is formulated as a liquid dosage form for oral administration and comprises:
(i) primidone or a pharmaceutically acceptable active metabolite, derivative, salt or solvate thereof in an amount of 250 mg to 2500 mg; and
(ii) a low-molecular weight PEG having an average molecular weight of about 180-420 g/mol, and preferably about 280-320 g/mol.

In another preferred embodiment, the pharmaceutical composition of the invention is formulated as a liquid dosage form for oral administration and comprises:
(i) primidone or a pharmaceutically acceptable active metabolite, derivative, salt or solvate thereof in an amount of 50 mg to 5000 mg;
(ii) a low-molecular weight PEG having an average molecular weight of about 180-420 g/mol, and preferably about 280-320 g/mol; and
(iii) an aqueous pharmaceutical carrier.

In another preferred embodiment, the pharmaceutical composition of the invention is formulated as a liquid dosage form for oral administration and comprises:
(i) primidone or a pharmaceutically acceptable active metabolite, derivative, salt or solvate thereof in an amount of 250 mg to 2500 mg;
(ii) a low-molecular weight PEG having an average molecular weight of about 180-420 g/mol, and preferably about 280-320 g/mol; and
(iii) an aqueous pharmaceutical carrier.

In another preferred embodiment, the pharmaceutical composition of the invention is formulated as a liquid dosage form for parenteral administration and comprises:
(i) primidone or a pharmaceutically acceptable active metabolite, derivative, salt or solvate thereof in an amount of 50 mg to 5000 mg;
(ii) a low-molecular weight PEG having an average molecular weight of about 280-320 g/mol; and
(iii) an aqueous pharmaceutical carrier,
wherein the PEG is present in the composition in an amount of 1-50%, more preferably 1-20% (w/w), based on the total weight of the composition.

In another preferred embodiment, the pharmaceutical composition of the invention is formulated as a liquid dosage form for parenteral administration and comprises:
(i) primidone or a pharmaceutically acceptable active metabolite, derivative, salt or solvate thereof in an amount of 250 mg to 2500 mg;
(ii) a low-molecular weight PEG having an average molecular weight of about 280-320 g/mol; and
(iii) an aqueous pharmaceutical carrier,
wherein the PEG is present in the composition in an amount of 1-50%, more preferably 1-20% (w/w), based on the total weight of the composition.

In another preferred embodiment, the pharmaceutical composition of the invention is formulated as a liquid dosage form for oral administration and comprises:
(i) primidone or a pharmaceutically acceptable active metabolite, derivative, salt or solvate thereof in an amount of 50 mg to 5000 mg;
(ii) a low-molecular weight PEG having an average molecular weight of about 280-320 g/mol; and
(iii) an aqueous pharmaceutical carrier,
wherein the PEG is present in the composition in an amount of 1-50%, more preferably 1-20% (w/w), based on the total weight of the composition.

In another preferred embodiment, the pharmaceutical composition of the invention is formulated as a liquid dosage form for oral administration and comprises:
(i) primidone or a pharmaceutically acceptable active metabolite, derivative, salt or solvate thereof in an amount of 250 mg to 2500 mg;
(ii) a low-molecular weight polyethylene glycol (PEG) having an average molecular weight of about 280-320 g/mol; and
(iii) an aqueous pharmaceutical carrier,
wherein the PEG is present in the composition in an amount of 1-50%, more preferably 1-20% (w/w), based on the total weight of the composition.

In another preferred embodiment, the pharmaceutical composition of the invention is formulated as a liquid dosage form for parenteral administration and comprises:
(i) primidone or a pharmaceutically acceptable active metabolite, derivative, salt or solvate thereof in an amount of 50 mg to 5000 mg;
(ii) a low-molecular weight PEG having an average molecular weight of about 280-320 g/mol; and
(iii) water as pharmaceutical carrier.

In another preferred embodiment, the pharmaceutical composition of the invention is formulated as a liquid dosage form for parenteral administration and comprises:
(i) primidone or a pharmaceutically acceptable active metabolite, derivative, salt or solvate thereof in an amount of 250 mg to 2500 mg;
(ii) a low-molecular weight PEG having an average molecular weight of about 280-320 g/mol; and
(iii) water as pharmaceutical carrier.

In another preferred embodiment, the pharmaceutical composition of the invention is formulated as a liquid dosage form for oral administration and comprises:
(i) primidone or a pharmaceutically acceptable active metabolite, derivative, salt or solvate thereof in an amount of 50 mg to 5000 mg;
(ii) a low-molecular weight PEG having an average molecular weight of about 280-320 g/mol; and
(iii) water as pharmaceutical carrier,
wherein the PEG is present in the composition in an amount of 1-50%, more preferably 1-20% (w/w), based on the total weight of the composition.

In another preferred embodiment, the pharmaceutical composition of the invention is formulated as a liquid dosage form for oral administration and comprises:
(i) primidone or a pharmaceutically acceptable active metabolite, derivative, salt or solvate thereof in an amount of 250 mg to 2500 mg;
(ii) a low-molecular weight PEG having an average molecular weight of about 280-320 g/mol; and
(iii) water as pharmaceutical carrier.

In another preferred embodiment, the pharmaceutical composition of the invention is formulated as a liquid dosage form for parenteral administration and comprises:
(i) primidone or a pharmaceutically acceptable active metabolite, derivative, salt or solvate thereof in an amount of 50 mg to 5000 mg;
(ii) a low-molecular weight PEG having an average molecular weight of about 280-320 g/mol; and
(iii) water as pharmaceutical carrier,
wherein the PEG is present in the composition in an amount of 1-50%, more preferably 1-20% (w/w), based on the total weight of the composition.

In another preferred embodiment, the pharmaceutical composition of the invention is formulated as a liquid dosage form for parenteral administration and comprises:
(i) primidone or a pharmaceutically acceptable active metabolite, derivative, salt or solvate thereof in an amount of 250 mg to 2500 mg;
(ii) a low-molecular weight PEG having an average molecular weight of about 280-320 g/mol; and
(iii) water as pharmaceutical carrier,
wherein the PEG is present in the composition in an amount of 1-50%, more preferably 1-20% (w/w), based on the total weight of the composition.

In another preferred embodiment, the pharmaceutical composition of the invention is formulated as a liquid dosage form for oral administration and comprises:
(i) primidone or a pharmaceutically acceptable active metabolite, derivative, salt or solvate thereof in an amount of 50 mg to 5000 mg;
(ii) a low-molecular weight PEG having an average molecular weight of about 280-320 g/mol; and
(iii) water as pharmaceutical carrier,
wherein the PEG is present in the composition in an amount of 1-50%, more preferably 1-20% (w/w), based on the total weight of the composition.

In another preferred embodiment, the pharmaceutical composition of the invention is formulated as a liquid dosage form for oral administration and comprises:
(i) primidone or a pharmaceutically acceptable active metabolite, derivative, salt or solvate thereof in an amount of 250 mg to 2500 mg;
(ii) a low-molecular weight PEG having an average molecular weight of about 280-320 g/mol; and
(iii) water as pharmaceutical carrier,
wherein the PEG is present in the composition in an amount of 1-50%, more preferably 1-20% (w/w), based on the total weight of the composition.

The therapeutic effectiveness of the pharmaceutical composition of the invention can be evaluated by using parameters known in the art. These parameters comprise, amongst others, the effectiveness of the composition according to the invention in eradicating symptoms of the treated disease, the response rate, the time until progression of the disease and the survival rate of the treated patients. Preferably, the compositions of the invention lead to a complete response in the patient. As used herein, complete response means the elimination of all clinically detectable disease symptoms as well as the restoration of normal results in blood count, x-ray examination, CT pictures, and the like. Such a response lasts preferably at least one month, and more preferably at least two or three months after the treatment has been stopped. The compositions of the invention can also result in a partial response in the patient. In a partial response, only some of the clinically detectable disease symptoms are reduced in the patient.

As indicated above, the medical use of primidone and its active metabolites, derivatives, salts and solvates has been contemplated for treating a disease or condition that involves a pathologic level or activation of RIPK1-dependent cell death. It was reported that these compounds effectively inhibit RIPK1 activity in cells and tissues. As such, primidone and its active metabolites, derivatives, salts and solvates are suitable active ingredients for preventing or inhibiting RIPK1-dependent cell death, in particular apoptosis and necroptosis, in numerous diseases and conditions, including ischemia-reperfusion injury, neurodegenerative diseases like Parkinson's or Alzheimer's disease and sepsis. Thus, in another aspect the present invention relates to a pharmaceutical composition as described above for use in a method of treating a disease or condition that involves a pathologic level of RIPK1-dependent cell death.

In one preferred embodiment, the disease to be treated by the pharmaceutical composition of the invention is selected from the group consisting of reperfusion injury disease, a systemic inflammatory disease, a transplantation-related disease, a neurodegenerative disease, an autoimmune disease, an ophthalmologic disease, and a pulmonary disease.

In one embodiment, the disease or condition to be treated by the pharmaceutical composition of the invention is a reperfusion injury disease. As used herein, a "reperfusion injury disease" includes diseases and conditions that involve tissue damage caused by reperfusion, i.e. tissue damage that occurs when the blood supply to tissue is restored after a period of ischemia or lack of oxygen. Tissue damage occurring after reperfusion is known to involve RIPK1-dependent processes. The importance of RIPK1-dependent cell death following ischemia and reperfusion has been demonstrated in rodent models (Linkermann et al. (2012)). Accordingly, it has been suggested that blocking RIPK1 activity could be a viable strategy for reducing ischemia reperfusion-related cell death in reperfusion injuries of the brain, retina, heart, kidney, and liver.

In one embodiment, the reperfusion injury disease to be treated by the pharmaceutical composition of the invention is stroke. One of the main events occurring during ischemic brain stroke is cell death (Liu C. et al. (2017)). It is well-known that necroptosis mediated by RIPK and MLKL proteins contributes to brain injury after ischemic stroke. Therefore, primidone can effectively protect brain tissue from ischemic injury by blocking RIPK1-dependent necroptosis.

In another embodiment, the reperfusion injury disease to be treated by the pharmaceutical composition of the invention is myocardial infarction (heart attack). Regulated necrosis is one of the main forms of cardiomyocyte death in heart disease and heart failure. Especially RIPK1-mediated necroptosis has been implicated in the progression of myocardial infarction (Oerlemans MI, et al. (2012)). Therefore, RIPK1 might serve as a novel therapeutic target for prevention of myocardial ischemia-reperfusion injury.

In yet another embodiment, the reperfusion injury disease to be treated by the pharmaceutical composition of the invention is acute kidney failure or acute liver failure (Linkermann et al. (2012); Takemoto K. et al (2014)).

In another embodiment, the disease or condition to be treated by the pharmaceutical composition of the invention that involves a pathologic level of RIPK1-dependent cell death is a systemic inflammatory disease which is preferably selected from the group consisting of sepsis and systemic inflammatory response syndrome (SIRS). Cell death mechanisms play an important role in the pathogenesis of these diseases. For this reason, the use of RIPK1 inhibitors for blocking RIPK1-dependent cell death has been considered particularly useful for treating sepsis (Degterev A. et al. (2019); Zelic M. (2018)).

In yet another embodiment, the disease or condition to be treated by the pharmaceutical composition of the invention is a transplantation-related disease, such as graft-versus-host-disease. Cell death processes like apoptosis and necroptosis were found to contribute to organ failure during organ transplantation and in graft-versus-host-disease (Shi, S. et al. (2019), Falcon, C. et al. (2017); Kanou T. et al. (2018); Pavlosky A. (2014)).

In another preferred embodiment, the disease to be treated by the pharmaceutical composition of the invention that involves a pathologic level of RIPK1-dependent cell death is a neurodegenerative disease selected from Alzheimer's disease, Parkinson's disease, Huntington's disease, amyotrophic lateral sclerosis (ALS), traumatic brain injury, and multiple sclerosis (MS). It is well known that RIPK1-dependent necroptosis promotes cell death and neuroinflammation in the pathogenesis of these diseases (Yuan, J. et al. (2019)).

In yet another embodiment, the disease to be treated by the pharmaceutical composition of the invention is an autoimmune disease. RIPK1-dependent cell death was found to be involved in the pathomechanisms that result in autoimmune disease like ulcerative colitis, Crohn's disease, rheumatoid arthritis, autoimmune cardiomyopathy, autoimmune hepatitis, lupus erythematosus, Graves' disease, Guillain-Barre syndrome (GBS), Hashimoto's thyroiditis, idiopathic thrombocytopenic purpura, juvenile idiopathic arthritis, myasthenia gravis, pemphigus vulgaris, psoriasis, Reiter's syndrome, scleroderma, Sjögren's syndrome, vasculitis, vitiligo, and Wegener's granulomatosis (Degterev A. et al. (2019)).

In yet another embodiment, the disease to be treated by the pharmaceutical composition of the invention is an ophthalmologic disease, such as retinal degeneration or retinitis pigmentosa (Sato K. et al. (2013); Do YJ et al. (2017)).

In yet another embodiment, the disease to be treated by the pharmaceutical composition of the invention is a pulmonary disease, such as chronic obstructive pulmonary disease (COPD) or acute respiratory distress syndrome (ARDS). It is particularly preferred that the disease to be treated is ARDS, preferably virus-induced ARDS. ARDS is a life-threatening complication that is associated with acute damage to the lungs. In addition, it is often associated with multi-organ failure in SIRS. It may result from infection with a pulmonary virus, such as a pulmonary coronavirus (Khot and Nadkar (2020)). The recent COVID-19 outbreak has shown that many patients infected with Sars-CoV-2 develop ARDS, with a high number of fatal cases. It has been known in the art that RIPK1-dependent cell death plays an important role in the development of ARDS (Pan et al. 2016).

Moreover, it has been described in WO 2021/048162 A1 that a SARS-CoV-2 infection triggers RIPK1 activation in respiratory epithelial cells collected as throat smears of symptomatic patients who had tested positive for SARS-CoV-2 by PCR. Therefore, primidone or a pharmaceutically acceptable active metabolite, derivative, salt or solvate thereof can be effectively used for treating ARDS. In a particularly preferred embodiment, the disease to be treated by the pharmaceutical composition of the invention is coronavirus-induced ARDS, such as ARDS caused by Sars-CoV-2-.

In yet another embodiment, the disease to be treated by the pharmaceutical composition of the invention is a cancer disease resulting from a tumor that overexpresses RIPK1. For example, an upregulation of RIPK1 has been described in lung and pancreatic tumours (Gong Y. et al. (2019)). Thus, in a preferred aspect of the invention the disease to be treated by the pharmaceutical composition of the invention is a cancer disease resulting from a RIPK1-expressing lung or pancreatic tumor.

The pharmaceutical composition of the invention comprising primidone or one of its active metabolites, derivatives, salts or solvates and a low-molecular weight PEG can be used in a combination therapy approach, e.g. together with one or more other active ingredients. The one or more other one or more other active ingredients preferably comprise another inhibitor of apoptosis or necroptosis, like GSK3145095 of GlaxoSmithKline or DNL747 of Sanofi. The primidone compound and the additional active ingredient can be administered to the subject in the form of a single pharmaceutical composition comprising both active ingredients. Administration of such a pharmaceutical composition will then automatically result in the delivery of both active ingredients. Alternatively, the primidone compound and the additional active ingredient may also be administered separately from each other, i.e. in the form of two separate pharmaceutical compositions, one containing the primidone compound, and the other containing the additional active ingredient. The two separate compositions can be administered simultaneously or sequentially in either order to the same or different administration sites.

For example, a combination therapy with the above-mentioned active ingredients may begin at day 1 of a treatment period, and a first therapeutically effective dose of the primidone compound is administered at that day. A first therapeutically effective dose of the other active ingredient, such as another inhibitor of apoptosis or necroptosis, can be administered either on the same day, e.g. simultaneously or within about 30, 60, 90, 120, 150, 180, 210 or 240 minutes after administration of the primidone compound. Alternatively, the other active ingredients can be administered 2, 3, 4, 5, 6 or 7 days after administration of the primidone compound. A skilled person will readily be able to design administration regimens which are suitable for providing the maximum therapeutic effect.

As used herein, the term "about", when used in combination with a numerical value, means ± 10% of the recited value.

### BRIEF DESCRIPTION OF THE FIGURES

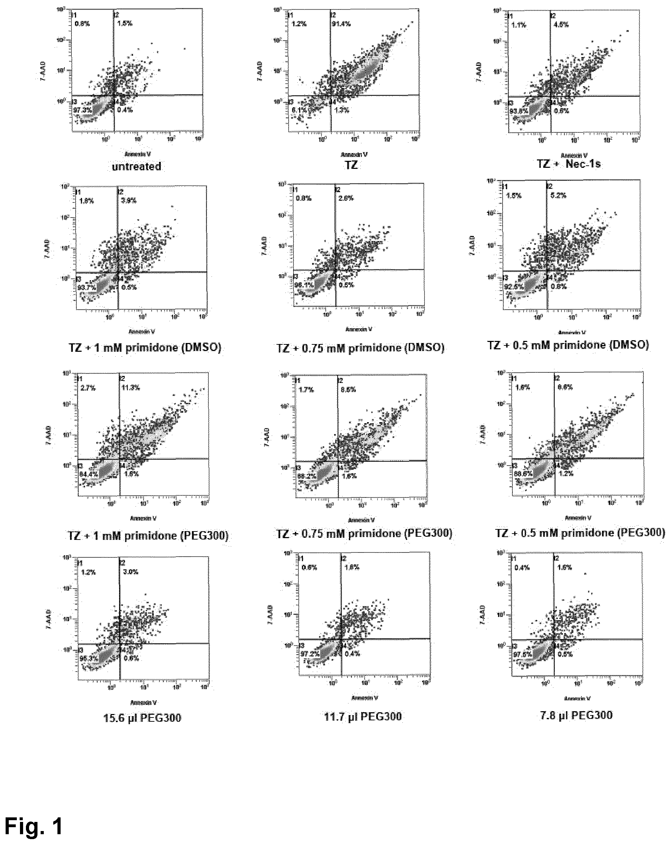

Fig. 1 shows the stimulation of NIH3T3 cells at 37°C for 24 h with 100 ng/ml TNFα (T) + 25 µM zVAD (Z) in the absence or presence of different concentrations (as indicated) of primidone, which was dissolved in DMSO and PEG300, respectively. The commercially available drug Necrostatin-1 (Nec-1s, 20 µM) acted as an internal control (inhibitor) of RIPK1-mediated cell death. All drugs were added 30 min before TNFα stimulation. RIPK1-mediated cell death was quantified by FACS analysis using 7-amino-actinomycin D and phosphatidylserine accessibility (Annexin V staining) as markers. Shown are data from one of three independent experiments.

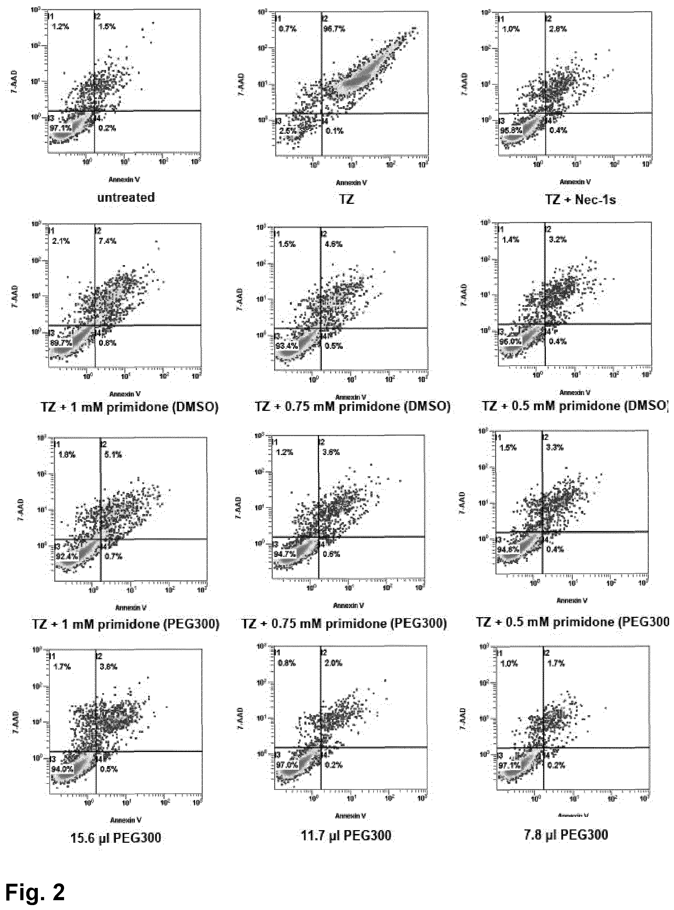

Fig. 2 shows the stimulation of L929 fibroblasts at 37°C for 24 h with 10 ng/ml TNFα (T) + 25 µM zVAD (Z) in the absence or presence of different concentrations (as indicated) of primidone, which was dissolved in DMSO and PEG300, respectively. The commercially available drug Necrostatin-1 (Nec-1s, 20 µM) acted as an internal control (inhibitor) of RIPK1-mediated cell death. All drugs were added 30 min before TNFα stimulation. RIPK1-mediated cell death was quantified by FACS analysis using 7-amino-actinomycin D and phosphatidylserine accessibility (Annexin V staining) as markers. Shown are data from one of three independent experiments.

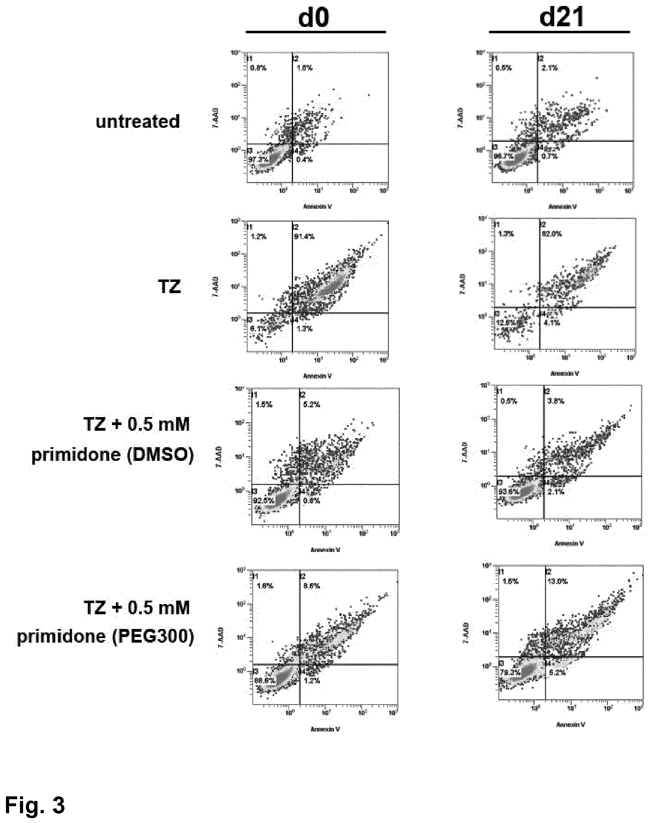

Fig. 3 shows the stimulation of NIH3T3 cells at 37 °C for 24 h with 100 ng/ml TNFα (T) + 25 µM zVAD (Z) in the absence or presence of 1 mM primidone, which was dissolved in DMSO or PEG300 three weeks ago and stored since that time darkened at room temperature (d21) in direct comparison with freshly prepared primidone (d0). All drugs were added 30 min before TNFα stimulation. Necroptotic cell death was quantified by FACS analysis using 7-amino-actinomycin D and phosphatidylserine accessibility (Annexin V staining) as markers.

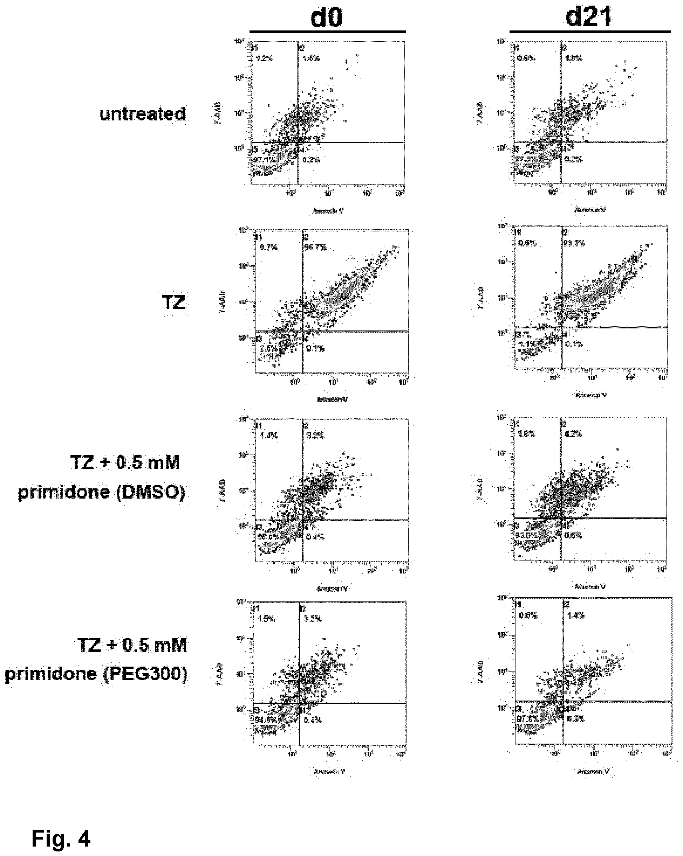

Fig. 4 shows the stimulation of L929 fibroblasts at 37 °C for 24 h with 10 ng/ml TNFα (T) + 25 µM zVAD (Z) in the absence or presence of 1 mM primidone, which was dissolved in DMSO or PEG300 three weeks ago and stored since that time darkened at room temperature (d21) in direct comparison with freshly prepared primidone (d0). All drugs were added 30 min before TNFα stimulation. Necroptotic cell death was quantified by FACS analysis using 7-amino-actinomycin D and phosphatidylserine accessibility (Annexin V staining) as markers.

### EXAMPLES

The present invention will be described in the following by preferred embodiments which merely illustrate the invention, but should by no means limit the invention.

### Example 1: Preparation of primidone test solutions

Briefly, 14 mg primidone were added to 1 ml PEG300 and dissolved overnight in a darkened vessel that was agitated. This gave rise to a 64.144 mM stock solution which was subsequently filtered sterile and stored at room temperature in the dark at room temperature until use. In parallel, a primidone stock solution of 64.144 mM was prepared with DSMO using the same conditions . The stock solution was also stored at room temperature. These stock solutions were used in the below cell experiments.

### Example 2: Testing the primidone solutions in cell-based assays

To measure whether primidone dissolved in either DMSO or PEG300 is able to protect cell from necroptotic cell death, NIH3T3 cells and L929 fibroblasts were treated with recombinant TNFα and the pan-caspase inhibitor zVAD to induce RIPK1-dependent necroptosis (Trichonas et al. 2010). The known RIPK1 kinase inhibitor Nec-1s was used as a control.

RIPK3-expressing NIH3T3 cells and L929 fibroblasts were obtained from the American Type Culture Collection (Manassas, VA, USA). Both cell lines were cultured in DMEM (Gibco, Thermo Fisher Scientific, Schwerte, Germany) supplemented with 10% (v/v) FCS (PAN-Biotech GmbH, Aidenbach, Germany), 100 U/ml penicillin, and 100 µg/ml streptomycin (both from Merck Millipore GmbH, Darmstadt, Germany). The cell lines were cultured in a humidified 5% CO₂ atmosphere at 37°C. Negativity for mycoplasma was routinely checked using a MycoAlert Mycoplasma Detection Kit (Lonza, Cologne, Germany). Unless stated otherwise, assays were conducted using 1x 10⁵ cells in 1 ml of medium.

Recombinant purified TNFα (# 570104) was purchased from BioLegend (Amsterdam, the Netherlands). The pan-caspase inhibitor zVAD-fmk (# 4026865) was purchased from Bachem (Weil am Rhein, Germany), Nec-1s (2263-1-BV) was obtained from BioCat (Heidelberg, Germany); primidone (# P7295) was purchased from Sigma-Aldrich Chemie GmbH (Taufkirchen, Germany). Polyethylenglycol (PEG300, # 202371) was purchased from Merck Millipore, Dimethyl sulfoxide (DMSO, A3672) was obtained from AppliChem (via Thermo Fisher Scientific).

For testing the ability of primidone to protect against RIPK1-driven cell death, cells were kept in 1 ml medium. To test 1 mM, 0.75 mM and 0.5 mM primidone in PEG300, we pipetted 15.6 µl, 11.7 µl and 7.8 µl, respectively, of the 64.144 mM stock solution to the 1 ml of the medium that contains the cells. To ensure that PEG300 is not cytotoxic per se at this concentration, the same volumes (15.6 µl, 11.7 µl and 7.8 µl) of the solvent PEG300 were tested without primidone on the cells (see bottom row of Figure 1). The test was repeated after 1 week, 2 weeks and 3 weeks of storage of the stock solutions at room temperature (data not shown).

Phosphatidylserine exposure to the outer cell membrane of apoptotic cells or inner plasma membrane of necrotic cells and incorporation of 7-amino-actinomycin D (7-AAD) into necrotic cells was quantified by flow cytometry analysis. After stimulation of cells under the indicated conditions, staining was performed on single-cell suspensions using FITC Annexin V.

Results: It was observed that equimolar concentrations of primidone were able to protect both NIH3T3 and L929 cells from RIPK1-mediated necroptosis, regardless of whether the drug was dissolved in DMSO or PEG300. The protective effects observed for primidone solubilized in PEG300 were as strong as those observed for Nec-1s.

### LITERATURE

Cho, Y.S. et al. Phosphorylation-driven assembly of the RIP1-RIP3 complex regulates programmed necrosis and virus-induced inflammation. Cell 137, 1112-1123 (2009).
Degterev A. et al. Targeting RIPK1 for the treatment of human diseases. Proc Natl Acad Sci USA, 116(20), 9714-9722 (2019).
Do YJ eta al. A novel RIPK1 inhibitor that prevents retinal degeneration in a rat glaucoma model. Exp Cell Res, 359(1):30-38 (2017).
Falcon, C. et al. Exploiting Cell Death Pathways for Inducible Cell Elimination to Modulate Graft-versus-Host-Disease. Biomedicines, 5(2), 30 (2017).
Gong Y. et al. The role of necroptosis in cancer biology and therapy. Mol Cancer. 18(1), 100 (2019).
Kanou T. et al. Inhibition of regulated necrosis attenuates receptor-interacting protein kinase 1-mediated ischemia-reperfusion injury after lung transplantation. J Heart Lung Transplant, 37(10), 1261-1270 (2018).
Khot WY and Nadkar MY The 2019 Novel Coronavirus Outbreak - A Global Threat. J. Assoc. Physicians India 68:67-71 (2020).
Linkermann A. et al. Rip1 (receptor-interacting protein kinase 1) mediates necroptosis and contributes to renal ischemia/reperfusion injury. Kidney Int, 81(8), 751-61 (2012).
Liu C. et al. Necroptosis: A novel manner of cell death, associated with stroke. Int J Mol Med, 41(2), 624-630 (2017).
Liu & Lalaoui. 25 years of research put RIPK1 in the clinic. Semin Cell Dev Biol, 109:86-95 (2021)
Oerlemans MI, et al. Inhibition of RIP1-dependent necrosis prevents adverse cardiac remodeling after myocardial ischemia-reperfusion in vivo. Basic Res Cardiol, 107(4), 270 (2012).
Pan L, et al. Activation of necroptosis in a rat model of acute respiratory distress syndrome induced by oleic acid. Acta Physiologica Sinica, 68(5): 661-668 (2016).
Pavlosky A. et al. RIPK3-mediated necroptosis regulates cardiac allograft rejection. Am J Transplant, 14(8), 1778-90 (2014).
Sato K. et al. Receptor interacting protein kinase-mediated necrosis contributes to cone and rod photoreceptor degeneration in the retina lacking interphotoreceptor retinoid-binding protein. J Neurosci, 33(44), 17458-68 (2013).
Shi, S. et al. Necroptotic Cell Death in Liver Transplantation and Underlying Diseases: Mechanisms and Clinical Perspective. Liver Transpl., 25(7), 1091-1104 (2019).
Sun, L. et al. Mixed lineage kinase domain-like protein mediates necrosis signaling downstream of RIP3 kinase. Cell 148, 213-227 (2012).
Takemoto K. et al. Necrostatin-1 protects against reactive oxygen species (ROS)-induced hepatotoxicity in acetaminophen-induced acute liver failure. FEBS Open Bio, 4, 777-87 (2014).
Trichonas et al. Receptor interacting protein kinases mediate retinal detachment-induced photoreceptor necrosis and compensate for inhibition of apoptosis. PNAS, 14, 107(50):21695-700 (2010).
Yuan, J. et al. Necroptosis and RIPK1-mediated neuroinflammation in CNS diseases. Nat Rev Neurosci, 20(1):19-33, (2019).
Zelic M. et al. RIP kinase 1-dependent endothelial necroptosis underlies systemic inflammatory response syndrome. J Clin Invest, 128(5), 2064-2075 (2018).

## Claims

1. Pharmaceutical composition comprising:
(i) primidone or a pharmaceutically acceptable active metabolite, derivative, salt or solvate thereof,
(ii) a low-molecular weight polyethylene glycol (PEG) having an average molecular weight of about 180-420 g/mol, and
(iii) optionally, an aqueous pharmaceutical carrier.

2. Pharmaceutical composition of claim 1, wherein said a low-molecular weight PEG has an average molecular weight of about 280-320 g/mol, and preferably an average molecular weight of about 300 g/mol.

3. Pharmaceutical composition of claim 1 or 2, wherein said composition comprises 50-5000 mg of primidone or the pharmaceutically acceptable active metabolite, derivative, salt or solvate thereof.

4. Pharmaceutical composition of any of claims 1-3, wherein PEG is present in the composition in an amount of 1-50% (w/w), based on the total weight of the composition.

5. Pharmaceutical composition of claim 4, wherein PEG is present in the composition in an amount of 1-20% (w/w), based on the total weight of the composition.

6. Pharmaceutical composition of any of claims 1-5, wherein said composition comprises at least one additional inhibitor of apoptosis or necroptosis.

7. Pharmaceutical composition of any of claims 1-6, wherein said composition is formulated for being administered by oral or parental administration.

8. Pharmaceutical composition of any of claims 1-7, wherein said composition is for use in a method of treating a disease that involves a pathologic level of RIPK1-dependent cell death.

9. Pharmaceutical composition for use of claim 8, wherein the disease is selected from the group consisting of a reperfusion injury disease, a systemic inflammatory disease, a transplantation-related disease, a neurodegenerative disease, an autoimmune disease, an ophthalmologic disease, and a pulmonary disease.

10. Pharmaceutical composition for use of any of claims 8-9, wherein the reperfusion injury disease is selected from myocardial infarction, stroke, acute kidney failure, and acute liver failure.

11. Pharmaceutical composition for use of any of claims 8-9, wherein the neurodegenerative disease is selected from Alzheimer's disease, Parkinson's disease, Huntington's disease, and amyotrophic lateral sclerosis (ALS), traumatic brain injury, and multiple sclerosis (MS).

12. Pharmaceutical composition for use of any of claims 8-9, wherein the autoimmune disease is selected from ulcerative colitis, Crohn's disease, rheumatoid arthritis, autoimmune cardiomyopathy, autoimmune hepatitis, lupus erythematosus, Graves' disease, Guillain-Barre syndrome (GBS), Hashimoto's thyroiditis, idiopathic thrombocytopenic purpura, juvenile idiopathic arthritis, myasthenia gravis, pemphigus vulgaris, psoriasis, Reiter's syndrome, scleroderma, Sjögren's syndrome, vasculitis, vitiligo, and Wegener's granulomatosis.

13. Pharmaceutical composition for use of any of claims 8-9, wherein the systemic inflammatory disease is selected from sepsis and systemic inflammatory response syndrome (SIRS) or wherein said pulmonary disease is chronic obstructive pulmonary disease (COPD) or acute respiratory distress syndrome (ARDS), in particular coronavirus-induced ARDS.

14. Pharmaceutical composition for use of any of claims 8-13, wherein said composition is to be administered in an amount of 1-20 mg of primidone or the pharmaceutically acceptable active metabolite, derivative, salt or solvate thereof per kg body weight of the patient.

15. Pharmaceutical composition for use of claim 14, wherein said composition is to be administered in an amount of 5-15 mg of primidone or the pharmaceutically acceptable active metabolite, derivative, salt or solvate thereof per kg body weight of the patient.
